# EUROPEAN PATENT APPLICATION

(11) **EP 0 607 986 A1**
(43) Date of publication of application: **27.07.1994**
(21) Application number: 94100863.3
(22) Date of filing: 21.01.1994
(51) Int. Cl.: A61F 13/56

(54) **Attachment systems for close fitting absorbent products**

(30) Priority: 22.01.1993 US 6578
(71) Applicant: McNEIL-PPC, INC., Milltown New Jersey 08850 (US)
(72) Inventor: Lichtstein, Bernard M., Elizabeth, NJ 07208 (US); Luchino, Thomas P., Freehold, NJ 07728 (US)
(74) Representative: Strehl Schübel-Hopf Groening & Partner

(57) **Abstract**

Absorbent articles having adhesive attachment means to secure the product to the part of the undergarment at the ends of the crotch region or beyond it generally have adhesive areas (12,14) that are created approximately in and/or attached to the anterior and posterior regions of the product, in particular, the anterior and posterior regions of the garment-facing surface (18) of the product. The central crotch region of such absorbent products, in particular, the central crotch region of the garment-facing surface of the product, is substantially free of adhesive. Attachment flaps (20,22) may be placed at the longitudinal sides or corner edges of such absorbent products.

## Description

### FIELD OF THE INVENTION

The present invention relates to absorbent products, especially those absorbent products that are attached to an undergarment by adhesive means.

### BACKGROUND OF THE INVENTION

Belted absorbent products have been almost universally supplanted by absorbent products having adhesive means for releasably attaching the products to an undergarment. A persistent problem with available adhesive attachment systems is that movement of the undergarment may translate into movement of the absorbent product. Such movement can result in misalignment with the source of fluid discharge thereby compromising the product's function as a fluid absorbent.

The recognition that product performance may be compromised by crushing and/or distortion of the product during wear has prompted a continuing effort to develop adhesive attachment systems that secure a product to the undergarment in a manner that minimizes unwanted movement of the product during its use.

For example, U.S. Patent 4,445,900, issued May 1, 1984, in the name of Robert Roeder, discloses a sanitary napkin having an adhesive pattern in the form of an "X" wherein the crossing portion of the "X" is located at the approximate center of the napkin. This pattern is said to eliminate the possibility of the adhesive lines attaching to each other during wear when the napkin becomes attached and reattached to the panty due to crushing and distortion.

U.S. Patent 4,605,405, issued August 12, 1986, in the name of Frederich Lassen, discloses a catamenial appliance having a positioning means for securing the appliance to the undergarment and permitting constrained longitudinal slidable movement in response to the wearer's motion. The positioning means comprises a strap in slidable contact with a fluid impermeable baffle. The strap is attached to the panty by means of adhesive patches at either end of the strap. In certain embodiments, a retention strap is attached transversely across the middle of the napkin on the baffle side to form a loop. The positioning means is passed through the loop before it is attached at both its ends to the panty. In use, the retention strap and napkin are said to slide over the surface of the panty attached positioning means to a degree dictated by the movement of the wearer's body relative to the panty.

U.S. Patent 4,609,373, issued September 2, 1986, in the name of Russell Johnson, discloses a coupling means for attaching a sanitary napkin to the crotch area of an undergarment. The coupling means comprises a tab, one end of which is attached to the pad and the other end of which is hanging free to be passed slidably through a loop attached to the crotch area of the panty. After the free end of the coupling means is passed through the loop attached to the panty, it is attached to the napkin thereby creating a loop coupling between the pad and the garment. The means is said to restrict the range of possible displacement of the pad while not transferring dynamic loads from the garment to the pad.

U.S. Patent 4,690,680, issued September 1, 1987, in the name of Maureen Higgins, discloses an absorbent article having adhesive attachment means consisting of an adhesive pattern having the shape of a block "I", the block "I" being defined by a base portion, a stem portion, and a head portion. The base and head adhesive portions each comprise an adhesive zone located in an end region of the absorbent article. The base and head adhesive portions each may comprise one or more strips of adhesive that extend either transversely across each end region or longitudinally along the article. The stem adhesive portion comprises a longitudinally extending zone of adhesive that is centrally located with respect to the longitudinal edges of the absorbent article. The stem adhesive portion preferably comprises one or more strips of adhesive or interrupted spaced lines of adhesive. This adhesive attachment means is said to minimize bunching and distortion of the product during its use.

U.S. Patent 4,758,241, issued July 19, 1988, in the name of Elissa Papajohn, discloses a sanitary napkin formed of a plurality of vertically stacked layers adhered together about their outer peripheral edge and including an elastomeric member along each of the longitudinal side edges of the napkin drawing the body into a cup shape. The shape provides a deep compartment for retaining fluid and an encircling wall against the flow of fluid from the compartment.

CH 643,730 A5 discloses a sanitary napkin having a trapezoidal anterior end, a posterior end that is trapezoidal with rounded corners, and a rectangular middle portion. Adhesive is placed in both of the end regions on the internal face to attach the napkin directly to the body.

Despite advances in adhesive attachment systems for absorbent products, currently available products continue to exhibit problems such as leakage attributed to unwanted movement or distortion of the absorbent article. Currently available products generally provide adhesive attachment means within the central crotch area where the adhesive attachment to the panty permits the panty movement to translate itself to movement of the absorbent product, thereby resulting in misalignment with the source of fluid discharge.

It is an object of this invention to provide an adhesive attachment system that secures an absorbent product, such as a sanitary napkin or incontinence product, to an undergarment, in a manner that decreases unwanted movement of the product during use. Generally, such products are secured to the relatively immobile and tight fitting part of the panty at the ends of the crotch region, or beyond it, where the crotch region is contiguous with the parts of the panty that begin to cover the abdomen and the buttocks.

### SUMMARY OF THE INVENTION

The present invention provides absorbent articles having adhesive attachment means to secure the products to the part of the undergarment at the ends of the crotch region or beyond it. Such products generally have adhesive areas that are created approximately in and/or attached to the anterior and posterior end regions of the product, in particular, the anterior and posterior end regions of the garment-facing side of the product. The central crotch region of the absorbent products of this invention, in particular, the central crotch region of the garment-facing side of the products, are generally substantially free of adhesive. Attachment flaps provided with adhesive attachment means may be placed at the longitudinal sides or corner edges of such absorbent products in addition to or in lieu of the adhesive areas.

More particularly, this invention relates to an absorbent article for adhesive attachment comprising a generally rectangular major portion having longitudinal sides and transverse ends, having a posterior end region, an anterior end region, and a central region therebetween, and having a garment-facing major surface, wherein adhesive attachment means are provided on said garment-facing major surface of said generally rectangular major portion solely on said anterior end region and said posterior end region. The article may further be provided with attachment flaps extending from the posterior and/or anterior end regions of the product.

In another embodiment, this invention relates to an absorbent article for adhesive attachment comprising a generally rectangular major portion having longitudinal sides and transverse ends, having a posterior end region, an anterior end region, and a central region therebetween; further comprising one or more attachment flaps extending from said posterior end region which attachment flaps have a garment-facing major surface; and further comprising adhesive attachment means provided solely on said garment-facing major surface of said attachment flaps.

### BRIEF DESCRIPTION OF THE DRAWINGS

Fig. 1 is an illustration of a sanitary napkin of this invention having adhesive areas in the posterior and anterior end regions and a small vertical strip of placement adhesive in the approximate center of the garment-facing side of the napkin.

Fig. 2 is an illustration of a sanitary napkin of this invention having adhesive areas and attachment flaps in the posterior and anterior end regions of the garment-facing side of the napkin and a small vertical strip of placement adhesive in the approximate center of the garment-facing side of the napkin.

Fig. 3 is an illustration of a sanitary napkin of this invention having adhesive areas and attachment flaps in the posterior and anterior end regions of the garment-facing side of the napkin.

Figure 4 is an illustration of a sanitary napkin of this invention having attachment flaps and two small vertical strips of placement adhesive along the approximate longitudinal axis of the center region of the garment-facing side of the napkin.

Fig. 5 is an illustration of a sanitary napkin of this invention having adhesive on the anterior and posterior regions of the garment-facing side of the napkin and attachment flaps at one end region of the napkin.

Fig. 5A is an illustration of a sanitary napkin of this invention having adhesive on the anterior and posterior regions of the garment-facing side of the napkin and attachment flaps at one end region of the napkin.

Fig. 6 is an illustration of a sanitary napkin of this invention having adhesive areas in the posterior and anterior end regions of the garment-facing side of the napkin and a small vertical strip of placement adhesive in the approximate center of the garment-facing side of the napkin.

### DETAILED DESCRIPTION OF THE INVENTION

It has been found that unwanted movement and displacement of an absorbent article can be decreased by strategically placing adhesive attachment means at or near the end portions of the product to secure the product to the part of the undergarment at the ends of the crotch region or beyond it. Many prior art absorbent articles have adhesive within the central crotch area where adhesive attachment to the panty permits panty movement to translate itself to movement of the absorbent product. This results in misalignment with the source of fluid discharge and early product failure. Indeed, wings which are centrally disposed on either side of an absorbent article and attach to the panty in the central crotch region can aggravate and amplify product movement and subsequent poor fit.

It has been found that it is preferable to uncouple attachment of the absorbent product from areas of the panty, such as the crotch region, that are in relatively high motion, and that are potentially loose, ill fitting panty areas that will pull the article away from the perineal region. The absorbent products of this invention are provided with adhesive and/or attachment flaps for securing the panty to the areas on either side of the crotch --posteriorly toward the buttocks and anteriorly toward the abdomen. Such attachment means decrease the product's susceptibility to intra-anatomic motions of the crotch or to the vagaries of loose or tight panty fit within the crotch area, thereby increasing the product's ability to maintain contact with the body and decreasing the degree of product movement relative to the body.

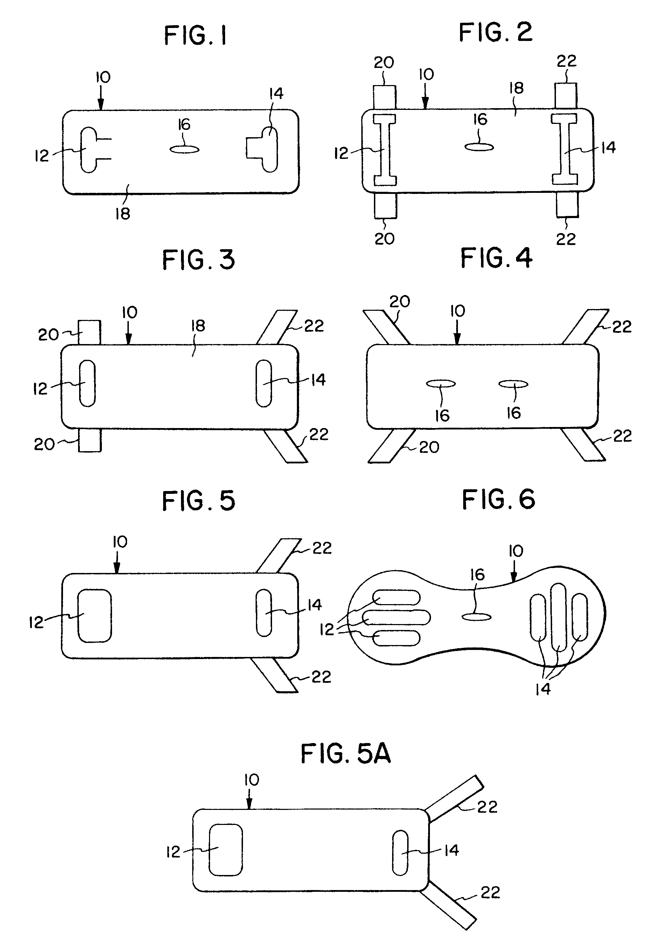

The absorbent products herein are products which absorb and contain body fluid, in particular, products that are placed in proximity to the body of the wearer. Such absorbent products may have any shape known in the art. A preferred embodiment of the absorbent product is a sanitary napkin, including pantyliners, although other absorbent products such as incontinence products are also contemplated as being with the scope of this invention. The sanitary napkin may have any shape known in the art. For example, Figs. 1-5A, illustrate a sanitary napkin having generally straight longitudinal edges with square or rounded ends. Fig. 6, illustrates a sanitary napkin having an hourglass shape wherein the longitudinal edges are curvilinear and the central region is narrower than the end regions. However, any design known to those skilled in the art can be used in the practice of the present invention.

Generally, the absorbent articles of this invention have a generally rectangular major portion having longitudinal sides and transverse ends. By generally rectangular it is meant that the longitudinal sides are of greater length than the transverse ends. Generally rectangular does not mean precisely rectangular and encompasses configurations such as dog-bone or hourglass shapes, and shapes with rounded or curved edges. The generally, rectangular major portion of the articles of this invention generally comprise a body facing cover, a means for absorbing body fluids, such as an absorbent core or batt, and a garment-facing surface (often a fluid impermeable backing sheet). The major portion has a posterior end region, an anterior end region, and a central region disposed therebetween. The central region is that area which is generally centrally located with respect to the ends of the article, below the perineum of the wearer. The anterior and posterior end regions extend outwardly from the central region toward the ends of the article. Although the exact size of the anterior and posterior end regions will vary according to the precise design and intended positioning of the article, in a preferred embodiment, each of the end regions comprises approximately one third of the total length of the article.

The body facing cover generally comprises a film or fabric having a high degree of moisture permeability. The absorbent core may be formed from any of the materials known to those of ordinary skill in the art to absorb body fluids, including, for example, cellulose wadding, wood pulp fibers, absorbent foams, superabsorbent polymers, or a combination of fluid absorbing materials. The garment-facing surface of the product is generally a moisture impermeable backing which may be any flexible, liquid impermeable material.

The body facing cover and fluid impermeable backing sheet are associated with the absorbent element by methods known in the art, including, for example, methods which employ adhesive to secure the cover and/or backing sheet to the absorbent element.

Disposed on the garment-facing surface of the major portion of the article is an adhesive attachment means. The attachment means are provided on the garment-facing surface solely on the anterior and posterior end regions to secure the product to the part of the undergarment at the ends of the crotch region or beyond it. In certain embodiments, at least one small area of positioning adhesive is provided in the approximate center and more preferably anterior of the center, of the central region of the garment-facing side of the product. The area of positioning adhesive serves solely to temporarily adhere the product to the undergarment as the product is being placed thereon. The area of placement adhesive is insufficient alone to hold the product in position during use and is therefore distinguishable from the adhesive attachment means provided on said anterior and posterior end regions. Generally, the area of positioning adhesive will comprise a total area no greater than about one square inches.

The pattern of the adhesive areas may take on various shapes and configurations, and may be solid or segmented, as long as they cover at least a portion of the posterior and anterior end regions of the article. Thus, for example, the adhesive shapes may be T's, inverted T's, ovals, circles, rectangles, squares, vertical or horizontal I's, vertical and/or horizontal and/or diagonal bars, and the like. The remaining central region is left substantially free of adhesive. However, a small spot or horizontal or vertical strip or area of adhesive may be placed in the approximate center and more preferably anterior of the center, of the central region to aid the initial placement of the article and to prevent the product from popping up in its middle as the user positions it on the panty.

The adhesive is generally, but not restricted to, a hotmelt formulation. Such adhesive is generally covered with a releasable covering that protects the exposed adhesive surface from contamination and from inadvertent sticking to itself and other surfaces before use, when the release covering is removed. Alternatively, the adhesive means may comprise pressure-sensitive adhesive tape, said tape having a first face permanently adhered to the garment-facing surface of the product and an opposite second face adapted to be temporarily attached to the garment. The adhesive may, alternatively, be placed on the body facing surface of the absorbent product if it is formulated to be sufficiently aggressive to hold on to the skin. Such adhesive is preferably comfortable to wear, hypoallergenic, atraumatic to remove, and does not leave adhesive residue behind.

While particular features of the absorbent articles of the present invention have been described, the articles may optionally be provided with additional elements known in the art. For example, flaps, wings or tabs (hereinafter referred to as "attachment flaps") provided with adhesive attachment means may be added to the sides or corners of the anterior and/or posterior end regions of the product. The attachment flaps can be made of and even be an extension of the components of the article, such as the body facing cover or fluid impervious backing, or they can be separately attached to the article. The adhesive attachment means provided on the attachment flaps and their adhesive releasable coverings are generally of similar composition and function to those described above. The attachment means are provided on the garment-facing major surface of the attachment flaps which (depending on how the flaps are to be used, e.g., whether they fold around an edge of the wearer's undergarment) may or may not be on the same side of the product as the garment-facing major surface of the generally rectangular major portion.

The attachment flaps may be placed at the longitudinal sides or corner edges of the article as extensions of the article edge, or attached somewhat inwardly of the article side or corner. They are preferably added to opposing sides along the anterior and posterior regions of the article and not at the anterior or posterior ends. Placing the attachment flaps at the sides or corners rather than the ends of the article prevents the article from moving from side to side, whereas placing them at the ends permits this movement to occur. The attachment flaps can be placed perpendicularly to the article's edge or corner or at an angle of between about 45° to 90°, up or down, to the longitudinal axis of the article.

The maximum length of the attachment flaps may be conveniently limited to that which can be wrapped around the end regions of the panty crotch or laid flat on the body facing inner panty surface. Attachment flaps placed at the posterior region of the article at an angle to the longitudinal axis of the article should, for practical reasons, not be longer than to overlay the parts of the panty covering the midpoints of the globes of the buttocks, which is an area particularly devoid of excessive motion when the user walks, sits or engages in other types of activities. The minimum length of such attachment flaps is generally about four inches.

Referring now to the Figures, Figure 1 is an illustration of a sanitary napkin **10** of this invention wherein the adhesive attachment means **12** and **14** comprise "T" shaped adhesive areas in the posterior and anterior end regions of the garment-facing side **18** of the product. The product illustrated in Figure 1 is further provided with a small vertical strip of placement adhesive **16**, preferably placed with the major portion of adhesive or all the adhesive being placed anterior of center.

Figure 2 is an illustration of a sanitary napkin **10** of this invention wherein the adhesive attachment means **12** and **14** comprise "I" shaped adhesive areas in the posterior and anterior end regions of the garment-facing side **18** of the product. The product illustrated in Figure 2 is further provided with a small area of placement adhesive **16** and with attachment flaps **20** and **22**.

Figure 3 is an illustration of a sanitary napkin **10** of this invention wherein the adhesive attachment means **12** and **14** comprise oval shaped adhesive areas in the posterior and anterior end regions of the garment-facing side **18** of the product. The product illustrated in Figure 3 is further provided with attachment flaps **20** and **22**.

For certain embodiments of the present invention, the attachment flaps may be used in lieu of adhesive attachment means in the anterior and posterior end regions of the napkin. Figure 4 illustrates an embodiment with attachment flaps **20** and **22** but without adhesive attachment means on the anterior and posterior end regions of the napkin **10**. The napkin in Figure 4 has the optional positioning adhesive **16** shown here as two small vertical strips. This embodiment is preferred for certain lightweight and/or streamlined or small napkin configurations wherein it is not necessary and even preferred not to have adhesive pattern areas when the attachment flaps **20** and **22** are employed.

Figures 5 and 5A, illustrate other embodiments of the present invention wherein, in addition to the attachment flaps **12** and **14**, one end of the napkin **10** has attachment flaps **22**. When only one end of the napkin has the attachment flaps, it is preferred that the flaps are placed at opposing sides or corners of the posterior region of the napkin where the major backward and forward, side to side, and distorting motions of the buttocks and the inner surfaces of the thighs occurs.

Figure 6 illustrates an hourglass shaped sanitary napkin **10** having adhesive attachment means **12** and **14** in the posterior and anterior end regions as well as a small vertical strip of positioning adhesive **16**.

Turning now to a more detailed description of the components of the articles of this invention, the body facing cover of the article generally comprises a film or fabric having a high degree of moisture permeability. Films made from hydrophobic bicomponent fibers, for example, polyester/polyester and polyester/polyethylene, are especially suitable. A typical hydrophobic bicomponent fiber has a polyester core and a polyethylene sheath. Preferably, the fabric used for the body facing cover is a lightweight fabric in the range of 0.3 to 5.0 oz. per square yard and with a density less than 0.2 gms/cc. The most suitable fabrics have unusually high elongation, loft, softness and drape characteristics. Films which are perforated or noncontinuous are also satisfactory. Though the cover is moisture permeable, it is preferably of the type which after permeation of the moisture, prevents strike-back of the body fluid when the absorbent structure is approaching saturation. The body facing cover is readily sealable to the outer rim of the moisture barrier, for example, by a heat seal or printed adhesive. The body facing cover may have different characteristics such as an embossed texture.

The means for absorbing body fluid may comprise any absorbent capable of absorbing human exudate. For example, this absorbent element may comprise a sheet of tenderized peat moss made by methods well known in the art. Generally, the raw peat moss material utilized is peat moss of the sphagnum variety and is preferably capable of absorbing at least about 15 times, preferably about 20 times, its weight in water. The peat moss is generally screened and then separated into a usable fraction and peat fines. The screened peat moss may be combined with other absorbent materials, preferably fibrous and cellulosic in nature. These art-recognized materials may include Kraft, wood pulp and mechanical wood pulp. This material is generally a chemically treated, long fibered pulp such as sulfite and sulfate wood pulps. A suitable mixture of ingredients for the absorbent elements of the invention may comprise from about 5 to about 20 percent by weight of Kraft wood pulp, with the remainder being essentially peat moss. Generally, the absorbent element is about 1/8 to 1/4 inch thick. It is understood that those familiar with the art may find a wide range of peat moss compositions as well as other absorbent materials for use with the products of this invention. Examples include but are not limited to blends of wood pulp fiber and superabsorbent materials, and laminates of wet-laid or air-laid pulp fabrics and superabsorbent.

The garment-facing surface of the product is generally a fluid impermeable barrier and may be any flexible, liquid impermeable material. Especially suitable are liquid-impermeable polyolefin films, e.g., polyethylene or polyethylene terephthalate. The backing sheet may be formed with materials and methods known in the art to prevent the fluids absorbed and contained in the absorbent core from wetting articles such as the undergarment which contacts the product.

While particular embodiments of the present invention have been illustrated and described, those skilled in the art will recognize that various changes and modifications can be made without departing from the spirit and scope of the invention.

## Claims

1. An absorbent article for adhesive attachment comprising a generally rectangular major portion having longitudinal sides and transverse ends, having a posterior end region, an anterior end region, and a central region therebetween, and having a garment-facing major surface, wherein adhesive attachment means are provided on said garment-facing major surface of said generally rectangular major portion solely on said anterior end region and said posterior end region.

2. The absorbent article of claim 1 wherein said article is provided with at least one small area of positioning adhesive in the approximate center to anterior of center of said central region of said garment-facing major surface.

3. The absorbent article of claim 1 wherein said adhesive areas comprise hotmelt adhesive.

4. The absorbent article of claim 1 wherein said adhesive areas are covered with a releasable covering.

5. An absorbent article of Claim 2 wherein said positioning adhesive comprises an area of adhesive no greater than about one square inch.

6. An absorbent article of Claim 1 further comprising one or more attachment flaps extending from said posterior end region, said attachment flaps having a garment-facing major surface, and adhesive attachment means being provided on said garment-facing major surface of said attachment flaps.

7. An absorbent article of Claim 1 further comprising one or more attachment flaps extending from said anterior end region, said attachment flaps having a garment-facing major surface, and adhesive attachment means being provided on said garment-facing major surface of said attachment flaps.

8. An absorbent article of Claim 6 further comprising one or more attachment flaps extending from said anterior end region, said attachment flaps having a garment-facing major surface, and adhesive attachment means being provided on said garment-facing major surface of said attachment flaps.

9. An absorbent article of Claim 6 wherein said attachment flaps extend from said longitudinal sides of said posterior end region.

10. An absorbent article of Claim 9 wherein said attachment flaps extend from said longitudinal sides of said posterior end region at an angle of between about 45° to 90° to said longitudinal sides.
